# EUROPEAN PATENT APPLICATION

(11) **EP 1 837 015 A1**
(43) Date of publication of application: **26.09.2007**
(21) Application number: 05819723.7
(22) Date of filing: 16.12.2005
(51) Int. Cl.: A61K 9/107, A61K 47/34, A61K 47/44

(54) **NOVEL CORE-SHELL STRUCTURE**

(30) Priority: 17.12.2004 JP 2004365667
(71) Applicant: Mitsubishi Chemical Corporation, Minato-ku Tokyo 108-0014 (JP)
(72) Inventor: ISOJIMA, T., Mitsubishi Chem. Group Science & Tech, Yokohama-shi, Kanagawa 2278502 (JP); ASATANI, H., Mitsubishi Chem. Group Sciece & Tech., Yokohama-shi, Kanagawa 2278502 (JP); SEKI, H., Mitsubishi Chem. Group Science& Techn., Yokohama-shi, Kanagawa 2278502 (JP); TAKEUCHI, H., Mitsubishi Chem. Group Science Tech., Yokohama-shi, Kanagawa 2278502 (JP)
(74) Representative: HOFFMANN EITLE
(86) International application number: PCT/JP2005/023548
(87) International publication number: WO 2006/073061

(57) **Abstract**

To provide a new structure-dispersed composite in which a substance poorly soluble in a medium is dispersed as micronized particles, a core-shell structure is dispersed in the medium, wherein said core-shell structure contains microparticles therein, and consists of a core comprising a core compound that is poorly soluble in said medium and a liquid shell comprising a shell compound that is poorly soluble in said medium, and the mean diameter of said core-shell structure is 10 µm or less.

## Description

### Technical Field

The present invention relates to a structure-dispersed composite, in which a core-shell structure is dispersed in a medium, said structure containing a core (central core), a shell (outer shell) and microparticles therein. The present invention also relates to a method for producing the composite, a core-shell structure to be used for the composite, and a drug product using the composite.

### Background Art

In various fields of industry and medicine, a composite is used in which a poorly soluble compound is dispersed in a liquid. For example, a compound poorly soluble in water is micronized and dispersed in water, or a compound poorly soluble in lipid is micronized and dispersed in lipid. In this way, drugs difficult to dissolve in water may be used for medical practice.

When a drug hardly soluble in water is administered to the human body as a dispersion in water, the drug is not easily absorbed into systemic circulation after administration because of its poor solubility, and it takes a long time until the drug exhibits its effect, or the drug is excreted from the body before it is absorbed into systemic circulation. Then, there is a possibility that the effect of the drug is not satisfactory. Recently, therefore, in the area of drug formulation, micronization of drugs has been extensively studied as a means of drug delivery of hardly water-soluble drugs for practical use. By micronizing drugs, stability of the dispersion of the drugs in water will increase, to thereby enhance absorption into the body and enhance effectiveness.

The method of micronization of this kind of hardly water-soluble drugs has been investigated in various ways. The methods investigated so far includes, for example: mechanically crushing and milling a hardly water-soluble large solid using surface-ameliorating agents such as surfactant; injecting a hardly water-soluble drug, dissolved in an organic solvent, using a spray; dissolving a hardly water-soluble drug in a water-soluble organic solvent and spreading in water allowing precipitation with poor solvent of the drug; dissolving a hardly water-soluble drug in a water-insoluble organic solvent, forming an emulsion with the aid of surfactant for example, and removing the water-insoluble organic solvent (in-liquid drying method); mechanically dispersing a hardly water-soluble drug in lipid, adding the dispersion to water forming an emulsion with the aid of surfactant, leading to drug-containing S/O/W type emulsion (Patent Documents 1 to 9) .

In Patent Document 10 is described, as core-shell structure, microparticles containing a drug in a shell. In that Patent Document, it is described that the above microparticles are produced by seed polymerization.
[Patent Document 1] US Patent No. 5145684
[Patent Document 2] Japanese Patent Application Laid-Open No. SHO 63-232840
[Patent Document 3] Japanese Patent Application Laid-Open No. SHO 57-27128
[Patent Document 4] Japanese Patent Application Laid-Open No. SHO 63-122620
[Patent Document 5] Japanese Patent Publication No. 3244502
[Patent Document 6] Japanese Patent Application Laid-Open No. HEI 1-156912
[Patent Document 7] Japanese Patent Application Laid-Open No. SHO 61-63613
[Patent Document 8] Japanese Patent Application Laid-Open No. HEI 4-46115
[Patent Document 9] Japanese Patent Application Laid-Open No. SHO 63-23811
[Patent Document 10] Japanese Patent Application Laid-Open No. HEI 7-53835

### Disclosure of the Invention

### Problem to Be Solved by the Invention

However, when poorly soluble substances are dispersed in a liquid as microparticles, further improvement in performance has been desired in various industry fields, depending on the specific use of the substance, and new technology has been sought to meet the various requirements. Take, for example, the above-mentioned drugs with poor water-solubility. By the previously known methods, a large amount of energy was required in micronization, the diameter of micronized drug particles was still not small enough, or the stability of drug formulation in water was not sufficient even if the diameter was small enough, and therefore there was room for further improvement. Further, according to the technology described in Patent Document 10, production of microparticles is effected by seed polymerization and, therefore, polymers used for microparticles are restricted to synthetic polymers, radicals are produced during the process of its production, leading to deterioration of drug quality, and drug release is not efficient because of polymer nature of the shell. These properties necessitated further improvement. Therefore, in the medical field, a method has been required which can process poorly water-soluble drugs into a form which has sufficiently small particle diameter and yet stable, in an inexpensive way.

The present invention has been made to solve the above problem. Namely, the purpose of the present invention is to provide a new structure-dispersed composite in which a substance poorly soluble in a medium is dispersed as micronized particles, a method for producing the composite, a core-shell structure to be used for the composite, and a drug product using the composite.

### Means for Solving the Problem

The present inventors made an intensive investigation to solve the above problem and have found a new structure-dispersed composite in which a core-shell structure is dispersed in a medium, wherein said core-shell structure contains microparticles therein, and consists of a core comprising a core compound that is poorly soluble in said medium and a shell comprising a liquid shell compound that is poorly soluble in said medium, and the mean diameter of said core-shell structure is 10 µm or less. These findings led to the completion of the present invention.

Namely, the subject matter of the present invention lies in a structure-dispersed composite in which a core-shell structure is dispersed in a medium, wherein said core-shell structure contains microparticles therein, and consists of a core comprising a core compound that is poorly soluble in said medium and a shell comprising a liquid shell compound that is poorly soluble in said medium and that can be phase-separated from the above core compound in said core-shell structure, and the mean diameter of said core-shell structure is 10 µm or less (claim 1). Through the use of the above composite, it is possible to disperse the microparticles in the medium in a hitherto unknown, new mode.

Another subject matter of the present invention lies in a method for producing the above structure-dispersed composite, comprising the step of: removing a non-miscible solvent, which is non-miscible with said medium, from emulsion in which liquid droplets are dispersed in said medium, said liquid droplets containing the above core compound, the above liquid shell compound, a specific compound constituting the above microparticles, and the non-miscible solvent being capable of dissolving the above core compound, liquid shell compound and specific compound (claim 9). By this method, it is possible to disperse the microparticles in the medium in a hitherto unknown, new mode.

Still another subject matter of the present invention lies in a core-shell structure comprising a core which forms the central core and a shell which forms the outer shell of said core, wherein said core comprises a core compound that is poorly soluble in water, said shell comprises a liquid shell compound that is poorly soluble in water and that can be phase-separated from the above core compound, said core-shell structure contains microparticles therein, and the mean diameter of said core-shell structure is 10 µm or less (claim 11). By this core-shell structure, it is possible to provide a structure with a hitherto unknown new, mode.

It is preferable that the microparticles are present at least in the shell (claim 2).

It is preferable that the mean diameter of the microparticles is 1 µm or less (claim 3).

It is further preferable that the microparticles are poorly soluble in the above liquid shell compound (claim 4).

It is further preferable that the microparticles are poorly soluble in said medium (claim 5).

It is further preferable that the above core compound is a biocompatible polymer compound (claim 6) .

It is preferable that said core-shell structure has a surfactant on the outer surface of the shell (claim 7).

Furthermore, it is preferable that the microparticles are a drug (claim 8).

Furthermore, it is preferable that the method for producing the above structure-dispersed composite further comprising the step of: micronizing the liquid droplets in the above emulsion (claim 10).

Another subject matter of the present invention lies in a drug product containing the above structure-dispersed composite (claim 12). Through the use of this composite, it will be easier to administer the drug product to treatment targets.

Still another subject matter of the present invention lies in a drug product containing the above core-shell structure (claim 13). Through the use of this structure also, it will be easier to administer the drug product to treatment targets

### Advantageous effect of the invention

According to the structure-dispersed composite and the method for its production of the present invention, it is possible to disperse microparticles in a medium in a hitherto unknown new mode.

The core-shell structure of the present invention provides a structure of a hitherto unknown new mode.

Furthermore, the drug product of the present invention makes possible administration of microparticles of drugs to treatment targets in an appropriate manner.

In addition, according to these invention, it is possible to maintain a specific compound, in particular a drug whose diameter is several tens nm, in a medium in a stable manner.

### Brief Description of the Drawings

[Fig. 1] Fig. 1(a) and Fig. 1 (b) are schematic drawings, illustrating the liquid droplet and the core-shell structure, as one embodiment of the present invention, before and after removal of non-miscible solvent. Fig. 1(a) illustrates the liquid droplet in the emulsion before removal of non-miscible solvent and Fig. 1(b) illustrates the core-shell structure after removal of non-miscible solvent.
[Fig. 2] Fig. 2 represents particle size distribution of the liquid droplets in the drug-containing emulsion A, measured in Example 1 of the present invention.
[Fig. 3] Fig. 3 represents particle size distribution of the core-shell structure in the drug-containing emulsion A after removal of chloroform, measured in Example 1 of the present invention.
[Fig. 4] Both Fig. 4(a) and Fig. 4(b) represent photos, which substitute for drawings, of the drug-containing emulsion A after removal of chloroform in Example 1, obtained by transmission electron microscopy.

### Best Modes for Carrying Out the Invention

The present invention will be explained below by referring to one embodiment. It is to be understood that the examples or the like shown below are by no means restrictive and any modifications can be added thereto insofar as they do not depart from the scope of the present invention.

### [I. Structure-dispersed composite]

The structure-dispersed composite of the present invention is a composite in which a core-shell structure is dispersed in a medium.

### [1. Medium]

The medium forms a system through which the core-shell structure of the present invention is dispersed. Any known compounds can be used as the medium insofar as the intent of the present invention is not significantly impaired. Usually, the structure-dispersed composite of the present invention is produced and used as a composite in which the core-shell structure of the present invention is dispersed in liquid. Therefore, it is preferable that the medium is a compound that can be liquid at the time of production and use. Concretely, the melting point of the medium is usually 200 °C or lower, preferably 100 °C or lower, more preferably 80 °C or lower. There is no lower limit to the melting point, but usually it is -200 °C or higher.

Furthermore, it is preferable to select the medium, taking into consideration the characteristics of the specific compound constituting the microparticles. Concretely, when the medium is liquid, it is preferable that the microparticles are poorly soluble in the medium. One of the advantages of the present invention is that it enables the microparticles of the specific compound, poorly soluble in a medium, to remain in the medium in a stable manner. Therefore, it is preferable to use a medium in which the specific compound is poorly soluble or insoluble, in order to make the best possible use of this advantage. In this specification, the term poor solubility means not only that a solute is poorly soluble in a medium but that a solute is totally insoluble in a medium. More concretely, the term includes such cases in which a hydrophobic substance is poorly soluble in hydrophilic solvent and hydrophilic substance is poorly soluble in hydrophobic solvent.

There is no special limitation on the state of existence of the medium insofar as the intent of the present invention is not significantly impaired. If water is taken up as an example of the medium, it may be in a liquid state at the time of use and in a state of frozen ice at the time of storage. The scope of the right of the present invention is not affected by the state of existence of the medium, and the composite in any state is the structure-dispersed composite of the present invention. In case resinous dispersion or the like is used as the medium, when it is allowed to solidify, the structure-dispersed composite of the present invention using a medium in a solid state becomes available.

As concrete examples of the medium can be cited hydrophilic solvents such as water, alcohol, tetrahydrofuran (THF) and methylethyl ketone (MEK); hydrophobic solvents such as ether, toluene and chloroform; resinous compounds such as polystyrene, polymethyl metacrylate, polyethylene and polyolefin. Of these, preferable is water. The medium can be used either as a single one or as a mixture of more than one kind in any mixing ratio. However, when two or more kinds of media are used, it is preferable that these media are miscible with each other at least when they are in a liquid state.

### [2. Core-shell structure]

The core-shell structure contained in the structure-dispersed composite of the present invention (hereinafter referred to as "core-shell structure of the present invention", as appropriate) comprises a core, which forms the central core, and a shell, which forms the outer shell of the core. Further, the core-shell structure of the present invention contains microparticles in it.

### [i. Core]

The core forms the central core of the core-shell structure of the present invention and comprises a core compound.

The core compound forming the core may be any compound poorly soluble in the liquid medium insofar as the intent of the present invention is not significantly impaired. A core compound poorly soluble in the medium is used because it is then easy to produce the structure-dispersed composite and core-shell structure of the present invention, and because the stability of the structure-dispersed composite and core-shell structure of the present invention then becomes excellent.

That the core compound is poorly soluble in the medium means that the core compound does not dissolve in the medium to the extent that allows the formation of the core-shell structure of the present invention in the structure-dispersed composite of the present invention. In more concrete terms, it means that the solubility of the core compound in the liquid state medium, under conditions of normal temperature and normal pressure (namely, 25 °C, 1013 hPa), is usually 30 weight % or less, preferably 10 weight % or less, more preferably 5 weight % or less. There is no lower limit but theoretically, it is 0 weight %.

Furthermore, it is preferable to select as core compound a compound which can cause phase separation from the liquid shell compound forming a shell (hereinafter referred to as "shell compound" as appropriate) so that the core and the shell result in phase separation in the core-shell structure. In order to examine whether phase separation occurs or not, the same amount of core compound and shell compound (for example, 1 g each) are mixed (for example, mixed in a 5 cc vial container in an arbitrary manner) and observed for occurrence of phase separation at a temperature for the use of the structure-dispersed composite or core-shell structure of the present invention (for example, room temperature).

As will be described later, the shell compound of the present invention is in a liquid state. It is preferable that the core compound is poorly soluble in a shell compound in that state. More concretely, the solubility of the core compound in the shell compound, under conditions of normal temperature and normal pressure, is usually 500 g/Liter or less, preferably 300 g/Liter or less, more preferably 100 g/Liter or less. Under these conditions, the core compound and the shell compound does not mix with each other during the process of removal of the non-miscible solvent at the time of production, leading to greater micronization of the microparticles. Namely, through precipitation of the core compound in the emulsion, described later, by removal of the non-miscible solvent, the space in which the microparticles grow becomes limited and aggregation of nuclei of the microparticles is inhibited, making possible greater reduction in diameter of the microparticles. Furthermore, following precipitation of the core compound described above, the core formed by the precipitated core compound works to push microparticles to the shell and this is considered to contribute to localization of the microparticles in the shell. There is no lower limit to the solubility of the core compound in the shell compound, but theoretically it is 0 g/Liter.

The state of existence of the core compound is not limited insofar as the intent of the present invention is not significantly impaired. It may be liquid, solid or gas.

There is no special limitation on the molecular weight of the core compound, insofar as the intent of the present invention is not significantly impaired. For example, when a polymer is used as core compound, the weight-average molecular weight of the core compound is usually 1000 or higher, preferably 3000 or higher, more preferably 5000 or higher, and usually 1000000 or lower, preferably 700000 or lower, more preferably 500000 or lower. When a monomer or oligomer is used as core compound and they are allowed to polymerize in the presence of a bridging agent, the core compound forms a gel and its molecular weight may be infinite. The weight-average molecular weight of the core compounds is determined by GPC (gel permeation chromatography). The conditions of determination such as medium used or column used are similar to those used for the GPC determination of usual polymers.

When hydrophilic solvent, such as water, is used as medium, polymers or inorganic materials can be cited as examples of the core compound. Furthermore, monomers or oligomers of these polymers can also be used for the core compound. In these cases, such monomers or oligomers may be polymerized during or after production of the core-shell structure to make a polymer core.

When the composite or core-shell structure of the present invention is used for medical purposes, as preferable examples of a polymer, used as core compound, can be cited polymers that can be degraded in the body (so-called biodegradable polymer). Concrete examples thereof include aliphatic polyesters, poly-α-cyanoacrylic acid esters, polyamino acids and copolymers of maleic acid anhydride. Representative examples of aliphatic polyesters include a single species polymer, copolymer, or a mixture of single species polymer and/or copolymer of α-hydroxyl carboxylic acids such as glycolic acid, lactic acid, 2-hydroxy butyric acid, 2-hydroxy valeric acid, 2-hydroxy-3-methyl butyric acid, 2-hydroxy caproic acid, 2-hydroxy isocaproic acid and 2-hydroxy caprylic acid, cyclic dimers of α-hydroxy carboxylic acid such as glycolide and lactide, hydroxyl dicarboxylic acids such as malic acid, hydroxyl tricarboxylic acids such as citric acid. As single species polymer can be cited a lactic acid polymer. As examples of copolymer can be cited a copolymer of lactic acid / glycolic acid, and a copolymer of 2-hydroxy butyric acid / glycolic acid. As a mixture of single species polymer and/or copolymer can be cited a mixture of lactic acid polymer and 2-hydroxy butyric acid / glycolic acid copolymer.

As examples of polyamino acids can be cited poly-γ-benzyl-L-glutamic acid, poly-L-alanine and poly-γ- methyl-L-glutamic acid.

As example of copolymer of maleic acid anhydride can be cited styrene / maleic acid copolymer.

Of these, preferable are aliphatic polyesters. Of aliphatic polyesters, particularly preferable are single species polymer, copolymer of two or more kinds, or a mixture of single species polymer and/or copolymer of α-hydroxy carboxylic acids and cyclic dimers of α-hydroxy carboxylic acid. Particularly preferable are single species polymer, copolymer or a mixture of single species polymer and/or copolymer of α-hydroxy carboxylic acids.

When the above α-hydroxy carboxylic acids, cyclic dimers of α-hydroxy carboxylic acid, hydroxyl dicarboxylic acids and hydroxyl tricarboxylic acids have an optical-activity center in the molecule, any isomer of D-compound, L-compound and DL-compound can be used.

The above aliphatic polyesters can be prepared by any known production methods (for example, production method described in Japanese Patent Application Laid-Open No. SHO 61-28521). Type of polymerization may be random, block or graft.

Furthermore, biocompatible polymers can be preferably used. Examples include polystyrene, poly meta-acrylic acid, copolymer of acrylic acid and meta-acrylic acid, polyamino acid, dextran stearate, ethyl cellulose, acetyl cellulose, nitro cellulose, copolymer of maleic anhydride series, copolymer of ethylene vinyl acetate series, polyvinyl acetate, polyacryl amide, polyurethane and polyethylene.

When inorganic material is used as core compound, preferable examples include metal such as gold, silica, titanium oxide, clay and talc.

Of those mentioned above, it is preferable to use biocompatible high molecular compound polymers as the core compound.

The core compound can be used either singly or as a mixture of more than one kind in any combination and ratio.

There is no special limitation on the diameter of the core insofar as the intent of the present invention is not significantly impaired. It is usually 1 nm or larger, preferably 3 nm or larger, more preferably 5 nm or larger, and it is usually 10 µm or smaller, preferably 5 µm or smaller, more preferably 3 µm or smaller. If the diameter is below the lower limit of this range, formation and maintenance of the core-shell structure may be difficult. If the diameter exceeds the above limit, stability of the dispersion of the core-shell structure itself may be inadequate. The diameter of the core can be decided, for example, by the observation using an electron microscope.

### [ii. Shell]

The shell forms an outer shell surrounding the periphery of the central core of the core-shell structure of the present invention and is formed by the shell compound.

The shell compound here means widely a compound which is poorly soluble in the liquid state medium, and capable of forming the shell of the core-shell structure of the present invention as a result of phase separation from the above-mentioned core compound in the core-shell structure.

That the shell compound is poorly soluble in the medium means that the shell compound does not dissolve in the medium to the extent that the core-shell structure of the present invention can be formed in the structure-dispersed composite of the present invention. More concretely, the solubility of the shell compound in the liquid state medium, under the conditions of normal temperature and normal pressure, is usually 30 weight % or lower, preferably 10 weight % or lower, more preferably 5 weight % or lower. There is no lower limit but theoretically, the lower limit of the above solubility is 0 weight %.

Further, as described above, the core compound and the shell compound are usually selected so that the core and the shell are phase-separated in the core-shell structure. Accordingly, it is preferable to select a shell compound that can phase-separate from a core-forming core compound. In order to examine whether phase separation occurs or not, a method similar to the one described above for the core compound can be used.

In the present invention, the shell compound is not limited to one forming the core-shell structure in a liquid state. Even if the compound is a solid under the normal temperature and normal pressure, it can be used as "liquid shell compound" if it becomes a liquid at the time of use and/or production (including immediately after production), by the rise of operation temperature and the like, of the structure-dispersed composite of the present invention. In other words, it is possible to use, as liquid shell compound, a compound that can be liquefied from a solid state while maintaining the constitution of the core-shell structure. Liquid state is meant a state in which the shell compound exists at a temperature above its melting point and, therefore, maintains fluidity. The melting point of the liquid shell compound is usually 100 °C or lower, preferably 65 °C or lower, more preferably 42 °C or lower.

When the core-shell structure of the present invention is isolated from the medium and used in that isolated form, it is possible to convert the shell compound into a solid temporarily by a process such as temporary freezing of the shell compound, which has previously been a liquid.

As will be described later, the structure-dispersed composite and the core-shell structure of the present invention are produced via a step involving removal of the non-miscible solvent. It is preferable that the shell compound is not removed in this process of removing the non-miscible solvent or removed to a smaller extent of amount than the non-miscible solvent. Usually, removal of the above non-miscible solvent is by evaporation, and therefore, it is preferable that the shell compound has a higher boiling point than the non-miscible solvent in order not to be removed. Concretely, the boiling point of the shell compound is usually 40 °C or higher, preferably 100 °C or higher, more preferably 200 °C or higher. There is no upper limit to the boiling point of the shell compound but usually, it is 500 °C or lower.

There is no special limitation on the molecular weight of the shell compound insofar as the intent of the present invention is not significantly impaired. When a polymer is used as shell compound, the molecular weight is usually 10000 or lower, preferably 5000 or lower, more preferably 1000 or lower. When this upper limit is exceeded, the release of drugs, used as microparticles, tends to be slow. Further, when the molecular weight of the shell compound is too high, the viscosity of the shell becomes high and formation of the shell may become difficult. No particular limitation is imposed on the lower limit of the molecular weight. Usually, it is 50 or larger. When a polymer is used as shell compound, the weight-average molecular weight should fall within the above range.

When hydrophilic solvent such as water is used as medium, examples of the shell compound include medium chain or higher fatty acids and alkyl esters thereof such as vegetable oils represented by soy bean oil, sesame oil, olive oil and cotton seed oil, cacao fat, fatty acid triglyceride, fatty acid diester of propylene glycol, and linoleic acid, alkyl ester of lactic acid, aromatic monomer, alkyl ester of dicarboxylic acid, and silicone oil. As examples, which can be a solid at normal temperature and pressure but can be liquefied by means of higher temperature or the like, can be cited higher fatty acid such as myristic acid, or fat such as beef fat which contains myristic acid as main component.

Also applicable as shell compound are: hydrocarbon solvents such as hexane, heptane and isooctane; aromatic hydrocarbons such as benzene, toluene and xylene; hydrophobic ketones such as methyl isobutyl ketone; esters solvents such as ethyl acetate; halogen-containing solvents such as dichloromethane and chloroform; alcohols such as propanol, 1-butanol and 1-pentanol; ethers such as diethyl ether, dipropyl ether and benzyl ethyl ether.

The shell compound can be used either as a single one or as a mixture of more than one kind in any combination and ratio.

There is no special limitation on the thickness of the shell insofar as the intent of the present invention is not significantly impaired. Usually, it is 1 nm or greater, preferably 3 nm or greater, more preferably 5 nm or greater, and usually 5 µm or smaller, preferably 4 µm or smaller, more preferably 3 µm or smaller. When the thickness is below the lower limit in this range, the space in which the specific compound can exist may be too narrow and a sufficient amount of the specific compound may not be maintained. When the upper limit is exceeded, stability of the dispersion of the core-shell structure itself may be inadequate. The thickness of the shell can be decided, for example, by the observation using electron microscope.

### [iii. Microparticle]

Microparticles are particles existing in the core-shell structure of the present invention and composed of a specific compound.

The specific compound here indicates a material which constitutes the above microparticles. No particular limitation is imposed on the kind of the material insofar as the intent of the present invention is not significantly impaired. One of the advantages of the present invention is that the particle diameter of the microparticles of the specific compound can be made very small and they can be dispersed in the medium in a stable manner. Therefore, when a compound which has been difficult to be reduced in diameter and to have stability of dispersion by the previously known methods is used as material of the microparticles, the advantage of the present invention can be displayed fully. Crystalline compounds or compounds which are solid at normal temperature are particularly suited as the specific compound.

As example of a property of a specific compound, which made their micronization and stability of dispersion difficult, can be cited easy crystallizability. According to the present invention, it is possible to disperse in a stable manner even specific compounds with the above property by selecting constituents of the core-shell structure appropriately.

The use of a specific compound which makes the emulsion, in which liquid droplets contain this specific compounds, to be unstable can display the above advantage effectively. Conventionally, in such emulsion, specific compounds in the liquid droplets precipitated out with the passage of time. However, in the structure-dispersed composite and core-shell structure of the present invention, this precipitation is prevented by the presence of the core compound and shell compound. Accordingly, it is possible to disperse such specific compounds in a stable manner.

Furthermore, it is preferable that the microparticles are poorly soluble in the medium. Therefore, it is preferable that a compound poorly soluble in the medium is used as specific compound, which is the material of the microparticles. By the conventional art, it has been difficult to disperse poorly-soluble microparticles in the medium in a stable manner. Therefore, the advantage of the present invention can be displayed fully when a poorly-soluble compound is used as microparticles. That the specific compound is poorly soluble in the medium means that the specific compound does not dissolve in the medium to the extent that allows the formation of the core-shell structure of the present invention in the structure-dispersed composite of the present invention. Concretely, the solubility of the specific compound in the liquid medium, under conditions of normal temperature and normal pressure, should be usually 100 g/Liter or less, preferably 50 g/Liter or less, more preferably 10 g/Liter or less. There is no lower limit to the solubility but theoretically, it is 0 g/Liter or higher.

Furthermore, when a shell compound is in a liquid state, it is preferable that the microparticls are poorly soluble in that shell compound. Accordingly, it is preferable that a specific compound constituting the microparticles is poorly soluble in the medium. Concretely, the solubility of the specific compound in the shell compound, under conditions of normal temperature and normal pressure, should be usually 100 g/Liter or less, preferably 50 g/Liter or less, more preferably 10 g/Liter or less. Under the above conditions, the formation of the microparticles of a specific compound in the shell is guaranteed. There is no lower limit to the solubility but theoretically, it is 0 g/Liter or higher.

No particular limitation is imposed on the state of existence of the microparticles insofar as the intent of the present invention is not significantly impaired. At the time of use or production, it is preferable that the microparticles are solid particles, in order to make the best possible use of the above advantage. However, it is possible to execute the present invention even when the microparticles are temporarily in a melted, liquid state.

There is no special limitation on the location of the microparticles in the core-shell structure of the present invention. Usually, it is preferable that the microparticles are in the shell. The microparticles residing in the shell enable the core-shell structure of the present invention to display its particular effect advantageously. For example, when a drug is used as the microparticles, it is possible to localize the drug close to the surface of the structure, differently from previously known microcapsules or the like. Therefore, it is possible to increase absorption of the drug when the structure-dispersed composite or the core-shell structure of the present invention is administered to a body. The microparticles may partly exist in the core but it is preferable that at least a part of them are in the shell.

When hydrophilic solvents such as water is used as the medium, suitable specific compounds constituting the microparticles include organic compounds and inorganic compounds. Particularly suitable are materials constituting known drugs, inorganic compounds, pigments and dyes.

Materials used for drugs, which represent one example of the specific compounds, include known materials used for various known drug category such as: analgesic, anti-inflammatory, anthelmintic, antiarrythmia, antibiotics (including penicillins), anticoagulant, antihypotensive, antidiabetic, anticonvulsant, antihistamine, antihypertensive, antimuscarinic, antimycobacterium, antineoplastic, immunosuppressant, antithyroid, antivirus, antianxiety (hypnotic and nerve relaxant), astringent, adrenergic β-receptor blocker, blood preparation and plasma substitute, cardiotonic, contrast media, corticosteroid, anticough (antitussive and mucolytic), diagnostic, diagnostic imaging agent, diuretic, dopaminergic (anti-Parkinson), hemostatic, immunomodulator, lipid-modifier, muscle relaxant, parasympathomimetic, parathyroid-related calcitonin, bisphosphonates, prostaglandin, radioactive drug, sex hormone (includes steroids), antiallergy, stimulant, anorectic, sympathomimetic, thyroid drug, vasodilator and xanthines.

Listed below are preferable concrete examples: 17-α-pregno-2,4 diene-20-ino-[2,3-d]-isoxazole-17-ol (danazole), 5α,17α,-1'-(methylsulfonyl)-1'H pregno-20-ino-[3,2-c]-pyrazol-17-ol (steroid A), [6-methoxy-4-(1-methylethyl)-3-oxo-1,2-benzisothiazol-2(3H)-yl]methyl- 2,6-dichlorobenzoate-1,1-dioxide, 3-amino-1,2,4-benzotriaine-1,4-dioxide, hyposulfam, hyposulfan, camptotecin, acetaminophen, acetylsalicylic acid, amiodarone, colestyramine, colestipol, chromoline sodium, albuterol, sucralfate, sulfasalazine, minoxidil, temazepam, alprazolam, propoxyphene, olanophine, erythromycin, cyclosporine, acyclovir, gancyclovir, etopocide, mefaran, methotrexate, minoxanthrone, daunorubicin, megesterol, tamoxifen, medroxyprogesterone, nystatin, terbutaline, amphotericin B, aspirin, ibuprofen, naproxen, indomethacin, diclofenac, ketoprofen, flurbiprofen, diflomisal, ethyl-3,5-diacetoamide-2,4,6-triiodobenzoate, ethyl(3,5-bis(acetylamino)-2,4,6-triiodobenzoyloxy)acetate and ethyl-2-(3,5-bis(acetylamino)-2,4,6-triiodobenzoyloxy)acetate. Particularly preferable are naproxen and indomethacin. These drugs can be used as the microparticles.

Examples of inorganic compounds used as specific compound include metal such as gold, silica, titanium oxide, clay and talc. Thus, it is possible to use metal particles such as colloidal gold or inorganic microparticles as the microparticles.

Furthermore, examples of specific compounds include pigments as listed below: quinacridone pigment, quinacridonequinone pigment, dioxazine pigment, phthalocyanine pigment, anthrapyrimidine pigment, anthanthrone pigment, indanethrone pigment, flavanethrone pigment, perylene pigment, diketopyrrolopyrrole pigment, perynone pigment, quinophthalone pigment, anthraquinone pigment, thioindigo pigment, metal complex pigment, azomethine pigment and azo pigment. Of these, preferable examples are phthalocyanine pigment or diketopyrrolopyrrole pigment. Thus, pigment particles can be used as the microparticles.

Furthermore, examples of specific compounds include dyestuffs such as lipophilic dye, direct dye, acid dye, basic dye, azoic dye and reactive dye.

Other dyestuffs applicable include dyestuffs used for ordinary writing-recording liquid such as those listed below: coumarin dye, perylene dye, dicyanovinyl dye, azo dye (for example, pyridone azo dye, bis azo dye, tris azo dye, benzene azo dye, heterocyclic azo dye etc.), quinophthalone dye, aminopyrazole dye, methine dye, dicyanoimidazole dye, indoaniline dye and phthalocyanine dye. Of these, preferable examples are azo dye, phthalocyanine dye and anthraquinone dye. Thus, dye particles can be used as the microparticles.

The specific compound can be used either singly or as a mixture of more than one kind in any combination and in any ratio.

There is no special limitation on the particle diameter of the microparticles insofar as the intent of the present invention is not significantly impaired. It is usually 1 nm or larger, preferably 3 nm or larger, more preferably 5 nm or larger, and usually 1µm or smaller, preferably 500 nm or smaller, more preferably 100 nm or smaller. This microparticles are stable particles having a very small diameter, and because of its small diameter and high stability in the medium, various advantages are anticipated. If the diameter exceeds the above upper limit, the above advantages of the microparticles may not be displayed fully. The mean diameter of the microparticles can be measured by electron microscopic observation.

### [iv. Surfactant]

The core-shell structure of the present invention usually has a surfactant on its surface. The surfactant is instrumental in making the core-shell structure stable in the medium.

There is no special limitation on the kind of surfactant and any known surfactant can be used so long as it can maintain the core-shell structure of the present invention in the medium. For example, anionic surfactant, cationic surfactant and non-ionic surfactant can be used. Also applicable are polymers possessing surface active potential.

As examples of anionic surfactant can be cited dodecylsulfonic acid, dodecylbenzene sulfonate, decylbenzene sulfonate, undecylbenzene sulfonate, tridecylbenzene sulfonate, nonylbenzene sulfonate and their sodium, potassium and ammonium salt.

As examples of cationic surfactant can be cited cetyltrimethylammonium bromide, hexadecylpyridinium chloride and hexadecyltrimethylammonium chloride.

As examples of non-ionic surfactant can be cited polyvinyl alcohol and various commercially available products such as "Triton" (X-100, X-114, X-305, N-101) of Union Carbide Co., "Tween" (20, 40, 60, 80, 85) of ICI Co., "Brij" (35, 58, 76, 98) of ICI Co., "Nonidet" (P-40) of Shell Co., "Igepol" (CO530, C0630, C0720, CO730) of Rhone Poulenc Co. and "Pluonic F68" of Asahi Denka Co..

As polymers having surface active potential can be cited naturally-occurring marmolecules such as dextran, pectin, dextrin, chondroitin sulfate, hyaluronic acid, heparin, gum arabic, albumin, casein, gelatin and collagen, polyamino acids and synthesized artificial proteins. Also applicable are synthesized polymers having both hydrophilic group and hydrophobic group, such as styrene-acrylic acid copolymer.

As surfactant can also be used anion-reactive surfactant, cation-reactive surfactant and non-ionic reactive surfactant. The use of these reactive surfactants makes it possible to prepare a core-shell structure to which surfactant molecules are covalently bonded. These structures have an advantage that the core-shell structure can remain stable even when th.e bulk of surfactant, which has been released into the medium from the structure-dispersed composite of the present invention, has been removed. Of these reactive surfactants, preferable are those having ethylene unsaturated group as a reactive group, such as vinyl group, allyl group and (meta)acryloyl group.

As reactive surfactant, any known compound possessing surface active potential can be used insofar as it has the above-mentioned reactive group. Concrete examples include those described in Japanese Patent Application Laid-Open No. HEI 9-279073. Of those, preferable anionic surfactants are: alkylbenzene sulfonates such as lauryl (allylbenzene) sulfonate, laurylstyrene sulfonate, stearyl (allylbenzene) sulfonate and stearylstyrene sulfonate, and their polyethyleneoxide adducts; alkylsulfosuccinic acid esters such as laurylallylsulfosuccinic acid ester, laurylvinylsulfosuccinic acid ester, stearylallylsulfosuccinic acid ester and stearylvinylsulfosuccinic acid ester, and their polyethyleneoxide adducts; alkyl or alkenylsulfonates such as (meta)acryllaurylsulfonate and oleylsulfonate; alkyl or alkenylsulfates such as (meta)acrylstearylsulfate and oleylsulfate, and their polyethyleneoxide adducts. Preferable cationic surfactants are: quarternary ammonium salts such as lauryl triallylammonium chloride, stearyl triallylammonium chloride and distearyl diallylammonium chloride. Preferable non-ionic surfactants are: polyethyleneglycolalkyl or alkenylphenyl ethers such as polyethyleneglycoloctyl (allylphenyl) ether, polyethyleneglycolnonyl (allylphenyl) ether and polyethyleneglycololeylphenyl ether; glycerin fatty acid esters such as monostearic monoallyl glycerin and distearic monoallyl glycerin and their polyethyleneoxide adducts; sorbitan fatty acid esters such as monostearic acid monoallylsorbitan and tristearic acid monoallylsorbitan and their polyethyleneoxide adducts; (meta)acrylic acid polyethyleneoxide esters such as polyethyleneglycol mono(meta)acrylate and polyethyleneglycol di(meta)acrylate.

Of the above reactive surfactants, commercially available anionic surfactants are, for example, "Acuarone HS-10" (Daiichi Kogyo Seiyaku Co.), "Antox-MS-60", "RA-1000 Series", "Antox-MS-2N" (Nihon Nyukazai Co.), "Adecaria Soap SE-10N" (Asahi Denka Co.), "Terumuru S-180A" (Kao Co.), and "Ereminor JS-2" (Sanyo Kasei Co.). Commercially available cationic surfactants are, for example, "RF-751" (Nihon Nyukazai Co.). Non-ionic surfactants commercially available are, for example, "Adecaria Soap NE-10" (Asahi Denka Co.) and "Brenmar PE-200", "Brenmar PE-350", "Brenmar PE-400" (Nihon Yushi Co.).

The above surfactant can be used either singly or as a mixture of more than one kind in any combination and ratio.

### [v. Ratio of each component used]

There is no special limitation on the ratio of the core compound in the structure-dispersed composite of the present invention, insofar as the intent of the present invention is not significantly impaired. Usually, it is 0.01 g/Liter or more, preferably 0.1 g/Liter or more, more preferably 1 g/Liter or more, and usually 500 g/Liter or less, preferably 300 g/Liter or less, more preferably 100 g/Liter or less. When the ratio is below the above lower limit, it may be difficult to form the core-shell structure. When the above upper limit is exceeded, formation of emulsion, which is prepared in the process of the production, may not be guaranteed.

There is no special limitation either on the ratio of the shell compound in the structure-dispersed composite of the present invention, insofar as the intent of the present invention is not significantly impaired. Usually, the ratio is 0.01 g/Liter or more, preferably 0.1 g/Liter or more, more preferably 1 g/Liter or more, and usually 500 g/Liter or less, preferably 300 g/Liter or less, more preferably 100 g/Liter or less. When the ratio is below the above lower limit, it may be difficult to form the core-shell structure. When the above upper limit is exceeded, formation of the emulsion, which is prepared in the process of the production, may not be guaranteed.

There is no special limitation either on the ratio of the specific compound in the structure-dispersed composite of the present invention, insofar as the intent of the present invention is not significantly impaired. The ratio is usually 0.001 weight % or higher, preferably 0.005 weight % or higher, more preferably 0.01 weight % or higher. When the ratio is below the above range, the expected advantages of the specific compound may not be fully exhibited. There is no special limitation on the upper limit, but usually it is 50 weight % or lower.

When a surfactant is used, there is no special limitation on its amount in the structure-dispersed composite of the present invention, insofar as it is above the critical micelle concentration. It is usually 0.01 weight % or higher, preferably 0.05 weight % or higher, more preferably 0.1 weight % or higher. When the ratio is below the above lower limit, the emulsion, which is prepared in the process of the production, may not be stable. There is no special limitation on the upper limit, but usually it is 50 weight % or lower.

There is no special limitation on the ratio of core compound vs. shell compound insofar as the intent of the present invention is not significantly impaired. The ratio "weight of core compound / (weight of core compound + weight of shell compound)" is usually 1 weight % or higher, preferably 5 weight % or higher, more preferably 10 weight % or higher, and usually 99 weight % or lower, preferably 95 weight % or lower, more preferably 90 weight % or lower. When the ratio is below the above lower limit, the core-shell structure may not exist in a stable manner. The above requirement on the range applies also when the core-shell structure is isolated from the medium.

Furthermore, there is no special limitation on the ratio of the core compound in the core-shell structure insofar as the intent of the present invention is not significantly impaired. Usually, the ratio is 1 weight % or higher, preferably 5 weight % or higher, more preferably 10 weight % or higher, and usually 99 weight % or lower, preferably 95 weight % or lower, more preferably 90 weight % or lower. When the ratio is below the above lower limit, the core-shell structure may not exist in a stable manner. When the upper limit is exceeded, the microparticles may not exist in the shell in a stable manner.

Furthermore, there is no special limitation either on the ratio of the shell compound in the core-shell structure insofar as the intent of the present invention is not significantly impaired. Usually, the ratio is 1 weight % or higher, preferably 5 weight % or higher, more preferably 10 weight % or higher, and usually 99 weight % or lower, preferably 95 weight % or lower, more preferably 90 weight % or lower. When the ratio is below the above lower limit, the microparticles may not exist in the shell in a stable manner. When the upper limit is exceeded, the core-shell structure may not exist in a stable manner.

Furthermore, there is no special limitation either on the ratio of the microparticles in the core-shell structure insofar as the intent of the present invention is not significantly impaired. The ratio is usually 0.003 weight % or higher, preferably 0.01 weight % or higher, more preferably 0.03 weight % or higher. When the ratio is below the above lower limit, the effect expected for the specific compound may not be displayed. No particular limitation is imposed on the upper limit, but usually it is 90 weight % or lower.

When a surfactant is used, there is no special limitation either on the ratio of the surfactant attached to the surface of the core-shell structure insofar as the intent of the present invention is not significantly impaired. Usually, the ratio is 0.001 weight % or higher, preferably 0.005 weight % or higher, more preferably 0.01 weight % or higher, and usually 95 weight % or lower, preferably 90 weight % or lower, more preferably 85 weight % or lower. When the ratio is below the above lower limit, it may not be possible to disperse the core-shell structure in the medium in a stable manner. When the upper limit is exceeded, the effect expected for the specific compound may not be displayed sufficiently.

Usually, the composition of the core-shell structure remains unchanged when it is taken out of the medium. Therefore, the composition of the core-shell structure present in the structure-dispersed composite of the present invention is similar to its composition when it is taken out of the medium.

### [vi. Other constituent]

The structure-dispersed composite and the core-shell structure of the present invention may contain additional constituents. Therefore, constituents other than those described above may be contained in the medium, core and shell. No particular limitation is imposed on these additional constituents insofar as the intent of the present invention is not significantly impaired. These additional constituents may be contained also when the core-shell structure of the present invention is isolated from the structure-dispersed composite of the present invention.

Examples of constituents that may be contained in the medium include: chlorides such as KC1 and NaCl, buffer, pH adjusting agent, viscosity adjusting agent, coloring agent, preservative, fungicide, evaporation-preventing agent and perfume.

### [vii. Physicochemical property of the core-shell structure]

The structure-dispersed composite of the present invention is stable and, usually, the specific compound does not precipitate out on standing, while in previously known dispersion of microparticles, specific compound precipitated out with the passage of time. In more concrete terms, the structure-dispersed composite of the present invention is stable to the extent that the specific compound constituting the microparticles does not precipitate out during the period of usually 1 day or longer, preferably 2 weeks or longer, more preferably 1 month or longer, after allowing to stand under conditions of normal temperature and normal pressure.

The mean diameter of the core-shell structure of the present invention is usually 10 nm or larger, preferably 30 nm or larger, more preferably 50 nm or larger, and usually 10 µm or smaller, preferably 5 µm or smaller, more preferably 1 µm or smaller. The above mean diameter range applies not only to the core-shell structure in the structure-dispersed composite of the present invention but also to the core-shell structure separated from the structure-dispersed composite of the present invention. When the diameter of the core-shell structure of the present invention is below the above lower limit, the specific surface area of the core-shell structure becomes large, possibly requiring a larger amount of surfactant. On the other hand, when the upper limit is exceeded, the stability of the structure-dispersed composite of the present invention itself may become low. Furthermore, by making the diameter of the core-shell structure of the present invention small, its specific surface area can be made large, and when the structure-dispersed composite or the core-shell structure of the present invention is used for slow-release drug products, it is possible to effect the efficient release of microparticles inside.

The measurement of the diameter of particles such as core-shell structure or liquid droplets in the emulsion of the present invention to be described later can be carried out by the dynamic light scattering method using, for example, "FPAR-1000" (Otsuka Denshi Co.) or "Microtrack UPA" (Microtrack Co.). The measurement can also be done by laser diffractometry, using, for example, "LA920" (Horiba Seisakusho Co.). Electron microscopic measurement by use of scanning electron microscope (SEM) or transmission electron microscope (TEM) is also available. Of these methods, preferable is a measurement based on dynamic light scattering method.

### [II. Method of production]

The method for production of the structure-dispersed composite of the present invention (hereinafter referred to as "the method for production of the present invention", as appropriate) comprises a step of removing a non-miscible solvent from emulsion, in which liquid droplets are dispersed in the above-mentioned medium, the liquid droplets containing the above-mentiond core compound, shell compound, specific compound, and non-miscible solvent.

### [1. Preparation of emulsion]

The emulsion used in the method for production of the present invention consists of dispersion of liquid droplets in the medium, as described above. In the droplets are contained the above mentioned core compound, shell compound, specific compound and non-miscible solvent. Further, surfactant is usually contained in the emulsion.

The kind and amount of the core compound, shell compound, specific compound and surfactant are as described before.

As non-miscible solvent, preferable is one in which the core compound, shell compound and specific compound are soluble and which is non-miscible with the above medium.

That the above core compound, shell compound and specific compound are soluble in the non-miscible solvent means that the core compound, shell compound and specific compound are soluble to the extent that allows the production of the structure-dispersed composite and core-shell structure of the present invention. In more concrete terms, the solubility of the respective core compound, shell compound and specific compound in the non-miscible solvent in the liquid state is usually 0.01 weight % or higher, preferably 0.05 weight % or higher, more preferably 0.1 weight % or higher under conditions of normal temperature and normal pressure. On the other hand, that the non-miscible solvent is non-miscible with the medium means that phase separation occurs when the non-miscible solvent and medium are present together.

It is preferable that the non-miscible solvent is volatile from the standpoint of ease of its removal in its removal step described later. Therefore, the boiling point of the non-miscible solvent is preferably low. In concrete terms, the boiling point of the non-miscible solvent is usually 0 °C or higher, preferably 5 °C or higher, more preferably 10 °C or higher, and usually 300 °C or lower, preferably 250 °C or lower, more preferably 200 °C or lower.

In this connection, it is preferable that the boiling point of the non-miscible solvent is lower than that of the shell compound. Concretely, the difference between the boiling point of the non-miscible solvent and that of the shell compound is usually 1 °C or larger, preferably 3 °C or larger, more preferably 5 °C or larger. There is no upper limit to this difference, but usually it is 500 °C or less.

When hydrophilic solvent is used as the medium, concrete examples of non-miscible solvent include: halogenated hydrocarbons such as dichloromethane, chloroform, dichloroethane, trichloroethane and carbon tetrachloride; ethers such as ethyl ether and isopropyl ether; ketones such as acetone; fatty acid esters such as ethyl acetate and butyl acetate; aromatic hydrocarbons such as benzene, toluene and xylene; alcohols such as ethanol and methanol; acetonitrile. Of these, preferable are halogenated hydrocarbons, and chloroform is particularly preferable.

The non-miscible solvent can be used either as a single one or as a mixture of more than one kind in any combination and ratio.

No particular limitation is imposed on the amount of the non-miscible solvent used insofar as it allows the production of the structure-dispersed composite and the core-shell structure of the present invention. However, the ratio of "(weight of the specific compound + weight of the core compound + weight of the shell compound) / (weight of the specific compound + weight of the core compound + weight of the shell compound + weight of the non-miscible solvent)" is adjusted in such a way that it is usually 0.1 weight % or higher, preferably 1 weight % or higher, more preferably 3 weight % or higher, and is usually 50 weight % or lower, preferably 45 weight % or lower, more preferably 40 weight % or lower. If the ratio is below the above lower limit, the productivity may decline. If the upper limit is exceeded, the viscosity of the emulsion may increase in the production process, leading to the difficulty in emulsion formation.

On the other hand, in order to take notice of the relation between the amount of the non-miscible solvent and the medium, the weight ratio of liquid droplets in the weight of the whole emulsion, namely, "(weight of the specific compound + weight of the core compound + weight of the shell compound + weight of the non-miscible solvent) / total weight of the emulsion" is usually 0.5 weight % or higher, preferably 1 weight % or higher, more preferably 5 weight % or higher, and usually 50 weight % or lower, preferably 40 weight % or lower, more preferably 20 weight % or lower. If the ratio is below the above lower limit, the productivity may decline. If the upper limit is exceeded, the emulsion may not be formed in a stable manner in the production process.

In preparing the emulsion, the medium, core compound, shell compound, specific compound, non-miscible solvent, appropriately used surfactant and other components are mixed. The order of mixing can be arbitrary insofar as the above emulsion can be obtained. However, in order to obtain the above emulsion securely, it is preferable first to prepare a solution of the core compound, shell compound and specific compound in the non-miscible solvent (solution for liquid droplets) and a solution or dispersion of the surfactant in the medium (solution for medium), and then mix the two.

It is preferable to include a step of micronization after each component is mixed. In other words, it is preferable to micronize the liquid droplets in the emulsion and reduce the size of particle diameter of the liquid droplets. This will facilitate the formation of the emulsion.

There is no special limitation on the method of micronizing the liquid droplets and any known method such as stirring can be applied. For example, it is desirable to use such dispersing instruments as beads mill, roll mill, sand mill, paint shaker, ultrasonic dispersion equipment and microfluidizing equipment (Microfluidizer: registered trade mark). Of these, preferable is ultrasonic dispersion equipment. Particularly preferable is application of ultrasonic dispersion to the emulsion after mixing, which is quite effective in micronizing the liquid droplets in the emulsion.

When ultrasonic dispersion is effected, there is no special limitation on the power of the ultrasonic dispersion instrument used insofar as the intent of the present invention is not significantly impaired. Usually, it is preferable that the power is 10 W or higher and 6000 W or lower. The power outside this upper limit may be used. However, if the power is too high, it is possible that fragments of chips, used for ultrasonic dispersion, contaminate the emulsion.

No particular limitation is imposed on the frequency of ultrasonic wave when ultrasonic dispersion is effected, insofar as the micronization of the droplets can be effected. Usually, it is 1 kHz or higher, preferably 5 kHz or higher, more preferably 10 kHz or higher, and usually 3 MHz or lower, preferably 100 kHz or lower, more preferably 30 kHz or lower.

No particular limitation is imposed on the length of time of ultrasonic dispersion, insofar as the intent of the present invention is not significantly impaired. It depends on the amount of the emulsion subjected to ultrasonic dispersion. Usually, it is 1 second or longer, preferably 15 minutes or longer, and usually 72 hours or shorter, preferably 1 hour or shorter.

Further, no particular limitation is imposed on the temperature condition of ultrasonic dispersion insofar as the intent of the present invention is not significantly impaired. Usually, it is desirable to perform the treatment at a temperature close to normal temperature (25 °C). However, as it is likely that the temperature rises during the treatment, the process is usually performed in a water bath.

Incidentally, when ultrasonic dispersion is performed in the presence of water using halogenated hydrocarbons such as chloroform, carbon tetrachloride, methylene chloride or ethylene chloride as non-miscible solvent, hydrogen halide such as HCl may be produced, leading to lowering of pH of the medium of the emulsion. This phenomenon is observed when water, for example, is used as the medium. When pH of the medium is lowered, it is possible that the molecular structure of the specific compound forming the core-shell structure changes or the core-shell structure is not formed at all. Therefore, when micronization is effected by ultrasonic dispersion, it is preferable that buffer, pH adjusting agent or ion exchange resin is added to the medium before or during ultrasonic dispersion. By this procedure, the above-mentioned lowering of pH of the medium can be prevented.

There is no special limitation on buffer, pH adjusting agent or ion-exchange resin used to prevent the lowering of pH, insofar as the structure-dispersed composite and the core-shell structure of the present invention can be produced. Concrete examples of buffer include phosphate, carbonate, Tris {2-Amino-2-hydroxymethyl-1,3-propanediol, Tris (hydroxymethyl) aminomethane} and HEPES {2-[4-(2-hydroxyethyl)-1-piperazinyl] ethanesulfonic acid}. Examples of pH adjusting agent are alkaline substances such as NaOH.

No particular limitation is imposed on the pH of the medium in the emulsion, insofar as the structure-dispersed composite and the core-shell structure of the present invention can be produced. From the standpoint of preventing the change of molecular construction of the compound constituting the core-shell structure or guaranteeing the formation of core-shell structure, pH of the medium is usually 1.0 or higher, preferably 1.5 or higher, more preferably 2.0 or higher, and usually 12 or lower, preferably 11 or lower, more preferably 10 or lower.

There is no special limitation on the diameter of the liquid droplets of the emulsion obtained insofar as the intent of the present invention is not significantly impaired. Usually, it is 10 nm or larger, preferably 30 nm or larger, more preferably 50 nm or larger, and usually 200 µm or smaller, preferably 100 µm or smaller, more preferably 50 µm or smaller. When the diameter is below the lower limit, the specific surface area of the emulsion becomes too large, possibly necessitating too much surfactant. When the upper limit is exceeded, the stability of the emulsion may decrease.

### [2. Removal of non-miscible solvent]

After preparation of the emulsion, the non-miscible solvent is removed from that emulsion. In more detail, the non-miscible solvent contained in the liquid droplets of the emulsion is removed.

There is no special limitation on the method of removal of the non-miscible solvent and any known method can be applied. Usually, the removal is by drying.

When the non-miscible solvent is removed by drying, there is no special limitation on the conditions of drying such as temperature, pressure or length of drying time, and any condition is allowed insofar as the removal is possible. To cite a suitable drying condition, the temperature is usually 300 °C or lower, preferably 200 °C or lower, more preferably 100 °C or lower. If the upper limit is exceeded, the temperature may be above the boiling point of the medium and the compounds in the emulsion may decompose. There is no special limitation on the lower limit but usually, it is -200 °C or higher. The pressure is usually 100 kPa or lower, preferably 70 kPa or lower, more preferably 50 kPa or lower. No particular limitation exists on the lower end but usually, it is 1.0 x 10⁻² Pa or higher. The drying time is usually 1 min or longer, preferably 5 min or longer, more preferably 10 min or longer. No upper limit exists but it is usually 1 week or shorter. When the medium is dried together with the non-miscible solvent, the medium may be added to the emulsion, as appropriate, during the drying.

Further, in order to facilitate the removal of the non-miscible solvent, it is preferable to carry out the drying using an instrument for drying. For example, the non-miscible solvent can be evaporated off under normal pressure or under gradually reduced pressure using a propeller-type stirrer or magnetic stirrer, or it can be evaporated off using a rotary evaporator while adjusting the level of vacuum. Freeze drying can also be used. Of these methods, preferable is the use of a rotary evaporator.

Following removal of the non-miscible solvent, the core-shell structure of the present invention is formed in the medium. Namely, following removal of the non-miscible solvent from the liquid droplets in the emulsion, the core compound and the shell compound are phase-separated in the liquid droplets, leading to the formation of the core and the shell. In this situation, what compound in the liquid droplets forms the core and what compound forms the shell may depend on the level of hydrophilic nature of each compound. Generally, it also depends on the kind of surfactant used or the like.

The mechanism of formation of the core-shell structure, as inferred by the inventors of the present invention, will be explained here, with a polymer core compound as an example.

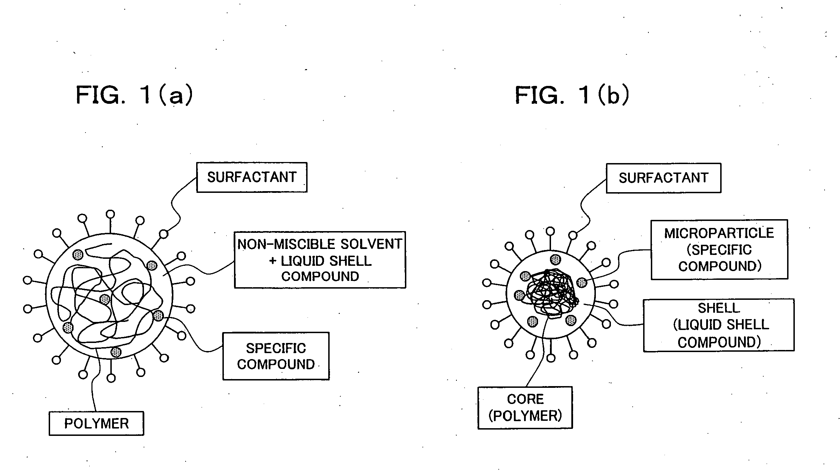

Fig. 1(a) and Fig. 1(b) are schematic drawings, illustrating the liquid droplet and the core-shell structure before and after removal of the non-miscible solvent. Fig. 1(a) illustrates the liquid droplet in the emulsion before removal of the non-miscible solvent and Fig. 1(b) illustrates the core-shell structure after removal of the non-miscible solvent. In Fig. 1(a), the polymer and the specific compound are illustrated for ease of explanation but, actually, these are dissolved in the non-miscible solvent and, therefore, invisible. Further, in Fig. 1(a) and Fig. 1(b), the surfactant molecules are illustrated but, as these surfactant molecules are very minute, they also are usually invisible.

As shown in Fig. 1(a), in the liquid droplets in the emulsion, the polymer, shell compound and microparticles are dissolved in the non-miscible solvent. When the non-miscible solvent is removed from the droplets, the polymer aggregates and precipitates out in the central portion of the droplets and the core is formed, as shown in Fig. 1(b). Around the core is formed a layer of the liquid shell compound, this layer becoming the shell. As the non-miscible solvent is removed, the specific compound precipitates out to form the microparticles. It is considered that these microparticles are pushed towards the shell by the precipitated polymer and, as a result, the microparticles are contained in the shell. By this mechanism, the core-shell structure of the present invention is formed and the structure-dispersed composite of the present invention is obtained.

Usually, surfactant molecules are attached to the periphery of the liquid droplets and the core-shell structure, and because of this, the liquid droplets and the core-shell structure are stable in the medium. With the removal of the non-miscible solvent, the volume corresponding to that removed non-miscible solvent decreases, and therefore, the diameter of the core-shell structure is usually smaller than that of the liquid droplet.

The structure of the above core-shell structure can be confirmed by, for example, electron microscopic observation of a ultrathin section.

### [3. Separation of core-shell structure]

The core-shell structure of the present invention may be used separately from the structure-dispersed composite of the present invention, as appropriate. No particular limitation is imposed on the concrete method of the separation and any method can be used insofar as it enables the separation of the core-shell structure. An example is to remove substances in the medium other than the core-shell structure by such means as centrifugation, ultrafiltration, gel filtration or dialysis, followed by removal of the medium by drying such as freeze drying or reduced pressure drying. Another method is to precipitate the core-shell structure by aggregation through salting out and remove the supernatant, leaving the precipitate.

### [4. Yield]

According to the production method of the present invention, usually 0.1 % or more, preferably 1 % or more, more preferably 3 % or more of the specific compound contained in the emulsion can be made to be the microparticles contained in the core-shell structure of the present invention. The upper limit of the yield is ideally 100 %. The yield can be analyzed by liquid chromatography, for example, after separation of the core-shell structure from the medium.

### [III. Application field]

The structure-dispersed composite and the core-shell structure of the present invention can be applied in various fields of industry, taking advantage of their new constitution. For example, when poorly water-soluble specific compound is made into microparticles, the core-shell structure can be prepared from poorly water-soluble core compound and poorly water-soluble shell compound. By including the microparticles in this core-shell structure, the structure-dispersed composite is obtained in which that core-shell structure is dispersed in water. It may be possible to achieve operation and effect, similarly to the structure-dispersed composite in which microparticles are dispersed in water. For example, when microparticles are constituted by a drug, it is possible to both micronize and disperse the drug in water in a stable manner even if the drug is poorly soluble in water. Therefore, it is possible to administer the drug appropriately as aqueous dispersion to the body which requires the drug treatment.

When the structure-dispersed composite and the core-shell structure of the present invention are used in medical field, it is preferable that each component of the structure-dispersed composite and core-shell structure, such as medium, core compound, shell compound, specific compound, surfactant and other component, is one approved for medical use. When a drug is used as microparticles, it is possible to use the structure-dispersed composite and core-shell structure of the present invention as additives for medical use.

As described above, when a drug component is used as the specific compound and microparticles, the structure-dispersed composite and the core-shell structure of the present invention promote absorption of the drug into the body because of its increased specific surface area, and make possible effective drug release by using a liquid shell compound. Further, the structure-dispersed composite and the core-shell structure of the present invention are excellent in stability of their dispersion and can display their advantage in drug delivery and storage. Further, application of the production method of the present invention to poorly water-soluble drugs provides a general method of micronizing drugs, which does not require much energy and is not affected by individual property of drugs.

Furthermore, the structure-dispersed composite and the core-shell structure of the present invention can be used in various areas in addition to the medical field mentioned above. For example, when pigments and dyes are used as the specific compound, they can be applied to ink for ink jet printer, tonner, resist for color filter and other ink or paint.

### [Example]

The present invention will be explained more concretely below by referring to examples. However, the present invention is not limited to these examples and any modification can be added thereto insofar as it does not depart from the scope of the present invention.

### [Example 1]

### (1) Preparation of emulsion

Amounts of 0.03 g of s-naproxen ((s)-(+)-6-Methoxy-α-methyl-2-naphthaleneacetic acid: Aldrich Co.), which is a drug (specific compound), 0.15 g of soy bean oil (Wako Pure Drug Co.), which is a liquid shell compound, and 0.3 g of poly-L-lactic acid (molecular weight 10000: Nakarai Tesque Co.), which is a core compound, were dissolved in 2.52 g of non-miscible solvent chloroform (Junsei Pure Chemical Co.). The solution was added to a 50 mL vial containing 0.3 g of SDS (sodium lauryl sulfate (surfactant): Nakarai Tesque Co.) and 26.7 g of desalted water (medium), and the mixture was dispersed using an ultrasonic disperser (ULTRA SONIC HOMOGENIZER UH-600S: STM Co.). The ultrasonic disperser was adjusted so that output chip was 7 ∅, output level was 5, interval level of ultrasonic irradiation was 50 %, and irradiation time was 15 minutes. During the irradiation, the vial was immersed in a water bath in order to prevent the rise in temperature. Thus, the drug-containing emulsion A was obtained as white turbid emulsion.

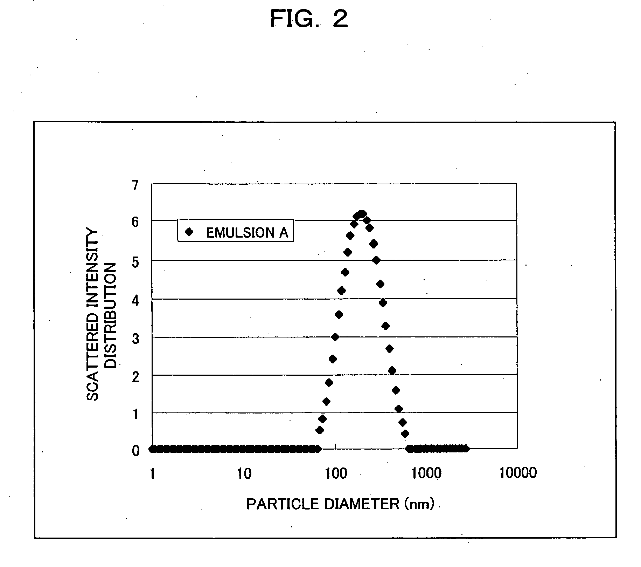

Particle size distribution of the drug-containing emulsion A obtained was measured by particle size distribution meter FPAR-1000 (Otsuka Denshi Co., probe for high concentration used). The result is shown in Fig. 2. The mean particle diameter was estimated to be about 180 nm.

### (2) Removal of chloroform from the emulsion

In order to remove chloroform contained in the droplets of the drug-containing emulsion A, 20 g of the emulsion was placed in a round-bottomed flask and the flask was immersed in a constant temperature bath, maintained at 60 °C. The chloroform was evaporated under carefully controlled reduced pressure, using a rotary evaporator, while care was taken to avoid bumping, until the weight of the emulsion decreased by more than the weight of the chloroform contained (1.68 g) and the odor of the chloroform was no longer perceptible. By his procedure, the chloroform was removed from the droplets of the drug-containing emulsion A and the chloroform-removed drug-containing emulsion A (structure-dispersed composite), in which the core-shell structure was dispersed in water, was obtained as white, turbid emulsion. The chloroform-removed drug-containing emulsion A obtained weighed 18.09 g.

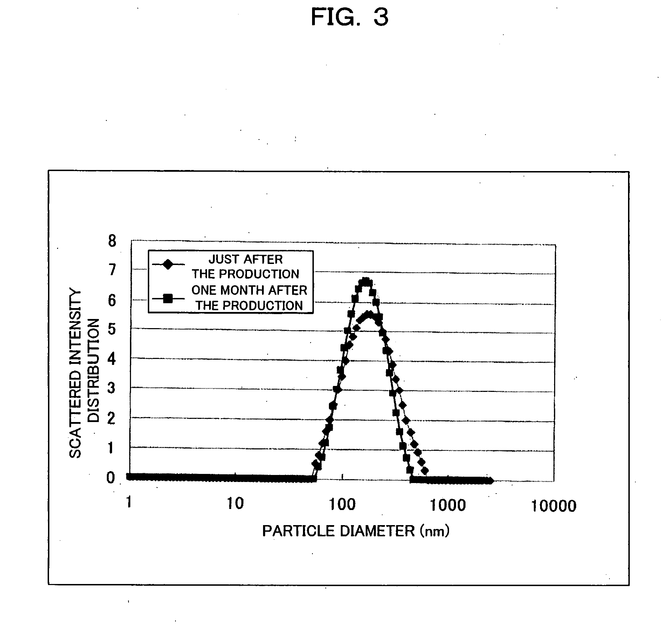

The distribution of particle size of the core-shell structure in the chloroform-removed drug-containing emulsion A was measured using FPAR-1000. The result is shown in Fig. 3. The mean particle diameter was found to be about 150 nm.

Similar measurement was repeated one month after removal of chloroform, the result of which is also shown in Fig. 3. The mean diameter of the core-shell structure was about 150 nm, demonstrating that no change occurred during storage.

From the above data, it was confirmed that the core-shell structure of the chloroform-removed drug-containing emulsion A is very stable.

### (3) Electron microscopic observation

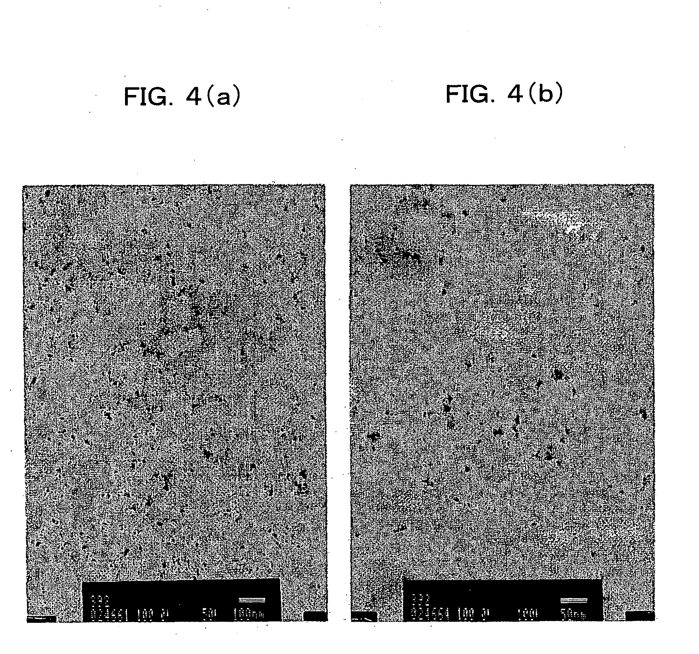

The chloroform-removed drug-containing emulsion A obtained was fixed with osmium tetroxide, embedded in epoxy resin, and ultrathin section was prepared using a ultramicrotome and the section was stained with uranium acetate / lead staining solution and observed with a transmission electron microscope (TEM). The photos are shown in Fig. 4(a) and Fig. 4(b). The two photos are for the same sample but with different magnification.

From the stained state and the amount of each reagent used, it is considered that areas of doughnut-like shadow (shell) in Fig. 4(a) and Fig. 4(b) are formed by soy bean oil, the inside (core) surrounded by the soy bean oil is formed by poly-L-lactic acid, and dots observed as deep shadow in the doughnuts represent s-naproxen. Accordingly, it was confirmed that the shell is formed by soy bean oil with poly-L-lactic acid as a core and the drug is microdispersed in the layer of the soy bean oil. It was also confirmed from electron microscopic photos that the particle diameter of s-naproxen is about 10 nm.

### [Example 2]

A drug-containing emulsion was prepared in the same manner as described in Example 1, except that the drug used was indomethacin (Indomethacin: Sigma Co.) and ultrasonic dispersion was performed for 30 minutes. This dispersion is called drug-containing emulsion B. The particle diameter of the core-shell structure of drug-containing emulsion B was measured by the same method as described in Example 1. The mean particle diameter was found to be about 150 nm.

Further, chloroform was removed from drug-containing emulsion B in the same manner as described in Example 1, and the mean particle diameter of this chloroform-removed drug-containing emulsion B (structure-dispersed composite) was measured by the same method as described in Example 1. The diameter was found to be about 110 nm. Repeated measurement after 2 weeks showed that the diameter was almost unchanged, being about 110 nm.

From these findings, it was confirmed that the core-shell structure in chloroform-removed drug-containing emulsion B is very stable.

### [Comparative example 1]

A drug-containing emulsion was prepared in the same manner as described in Example 1, except that soy bean oil and poly-L-lactic acid were not used and the amount of chloroform was 2.97 g. The drug-containing emulsion obtained was analyzed by FPAR-1000 and the mean diameter was found to be 600 nm. This emulsion separated into white precipitate and supernatant after 2 hours.

Further, chloroform was removed immediately after preparation of the drug-containing emulsion, similarly to Example 1. The emulsion after removal of chloroform was colorless and transparent, but many minute crystals, observed visually, separated out and precipitated about 1 hour later.

### [Comparative example 2]

A drug-containing emulsion was prepared in the same manner as described in Comparative Example 1, except that the drug used was indomethacin. The emulsion obtained separated into white precipitate and supernatant after about 20 minutes and, therefore, particle size distribution could not be measured.

Further, chloroform was removed immediately after preparation of the drug-containing emulsion, similarly to Example 1. The emulsion after removal of chloroform was colorless and transparent but many minute crystals, observed visually, separated out and precipitated about 1 hour later.

### [Summary]

From the comparison between Example 1, Example 2 and Comparative Example 1, Comparative Example 2, it has been confirmed that the core-shell structure in the chloroform-removed drug-containing emulsion A and B of Example 1 and Example 2 is very small in particle diameter and very stable. With respect to the drug-containing emulsion, which is an intermediate in the production process, it is evident that those of Example 1 and Example 2 are very stable, while those of Comparative Example 1 and Comparative Example 2 are unstable and separation occurs easily. It has now been confirmed that the chloroform-removed drug-containing emulsion A and B of Example 1 and Example 2 and the core-shell structure contained in it can be produced easily.

### Industrial Applicability

The present invention can be applied widely in various fields of industry. For example, it can be suitably applied for drug products, ink and paint.

The present invention has been explained in detail by referring to specific examples. However, it is evident for those skilled in the art that various modifications can be added thereto without departing from the spirit and scope of the present invention.

The present application is based on Japanese Patent Application (Patent Application No. 2004-365667) filed on December 17, 2004 and Japanese Patent Application (Patent Application No. 2005-362826) filed on December 16, 2005, and their entireties are incorporated by reference.

## Claims

1. A structure-dispersed composite in which a core-shell structure is dispersed in a medium, wherein
said core-shell structure contains microparticles therein, and consists of a core comprising a core compound that is poorly soluble in said medium and a shell comprising a liquid shell compound that is poorly soluble in said medium and that can be phase-separated from the above core compound in said core-shell structure, and
the mean diameter of said core-shell structure is 10 µm or less.

2. A structure-dispersed composite as defined in claim 1, wherein the microparticles are present at least in the shell.

3. A structure-dispersed composite as defined in claim 1 or claim 2, wherein the mean diameter of the microparticles is 1 µm or less.

4. A structure-dispersed composite as defined in any one of claims 1 to 3, wherein the microparticles are poorly soluble in the above liquid shell compound.

5. A structure-dispersed composite as defined in any one of claims 1 to 4, wherein the microparticles are poorly soluble in said medium.

6. A structure-dispersed composite as defined in any one of claims 1 to 5, wherein the above core compound is a biocompatible polymer compound.

7. A structure-dispersed composite as defined in any one of claims 1 to 6, wherein said core-shell structure has a surfactant on the outer surface of the shell.

8. A structure-dispersed composite as defined in any one of claims 1 to 7, wherein the microparticles are a drug.

9. A method for producing the structure-dispersed composite as defined in any one of claims 1 to 8, comprising the step of:
removing a non-miscible solvent, which is non-miscible with said medium, from emulsion in which liquid droplets are dispersed in said medium, said liquid droplets containing the above core compound, the above liquid shell compound, a specific compound constituting the above microparticles, and the non-miscible solvent being capable of dissolving the above core compound, liquid shell compound and specific compound.

10. A method for producing the structure-dispersed composite as defined in claim 9, further comprising the step of:
micronizing the liquid droplets in the above emulsion.

11. A core-shell structure comprising a core which forms the central core and a shell which forms the outer shell of said core, wherein
said core comprises a core compound that is poorly soluble in water,
said shell comprises a liquid shell compound that is poorly soluble in water and that can be phase-separated from the above core compound,
said core-shell structure contains microparticles therein, and
the mean diameter of said core-shell structure is 10 µm or less.

12. A drug product containing the structure-dispersed composite as defined in claim 8.

13. A drug product containing the core-shell structure as defined in claim 11.
